(19) Europäisches Patentamt
European Patent Office
Office européen des brevets

(11) Numéro de publication: **0 126 691 B1**

# (12) FASCICULE DE BREVET EUROPEEN

(45) Date de publication du fascicule du brevet: **20.04.88**

(51) Int. Cl.⁴: **A 61 K 35/78**, A 61 K 31/35

(21) Numéro de dépôt: **84401019.9**

(22) Date de dépôt: **17.05.84**

(54) **Procédé de préparation d'un extrait de Brackenridgea zanguebarica utile en thérapeutique, extrait et médicament correspondant.**

(30) Priorité: **18.05.83 FR 8308244**

(43) Date de publication de la demande: **28.11.84 Bulletin 84/48**

(45) Mention de la délivrance du brevet: **20.04.88 Bulletin 88/16**

(84) Etats contractants désignés: **AT BE CH DE FR GB IT LI LU NL SE**

(56) Documents cités:
**FR - A - 2 007 352**
**FR - A - 2 207 700**
**FR - A - 2 268 518**

**CHEMICAL ABSTRACTS, vol. 80, no. 21, 27 mai 1974, page 220, no. 118220b, Columbus, Ohio, US; E. BOMBARDELLI et al.: "Plants of Mozambique. IV. Flavonoids of Brackenridgea zanguebarica"**

(73) Titulaire: **INVERNI DELLA BEFFA S P A, Via Ripamonti, 99, I-20141 Milano (IT)**
Titulaire: **FOURNIER INNOVATION ET SYNERGIE, 38, avenue Hoche, F-75008 Paris (FR)**

(72) Inventeur: **Magistretti, Maria José, Via Tonale 9, I-20125 Milano (IT)**
Inventeur: **Reginault, Philippe, 13, rue de Provence, F-21121 Fontaine-les-Dijon (FR)**

(74) Mandataire: **Combe, André et al, CABINET BEAU DE LOMENIE 55, rue d'Amsterdam, F-75008 Paris (FR)**

**EP 0 126 691 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau procédé de préparation d'un extrait de Brackenridgea zanguebarica. Elle concerne également l'utilisation de cet extrait en thérapeutique notamment, d'une part, en tant qu'agent vasodilatateur dans le traitement de maladies de la circulation artérielle (en particulier la circulation artérielle périphérique et cérébrale) et, d'autre part, en tant qu'agent anti-agrégant plaquettaire et antithrombotique.

Brackenridgea zanguebarica est un arbre appartenant à la famille des Ochnaceae qui est répandu en Afrique australe et notamment au Mozambique. On sait que l'on a déjà préconisé en médecine populaire d'utiliser les feuilles de cette plante pour le traitement des blessures et des morsures de serpent et comme aphrodisiaque.

On sait de l'article de E. BOMBARDELLI et al., Phytochemistry 13, 295-297 (1974) que l'extrait hydroalcoolique total de Brackenridgea zanguebarica obtenu par extraction des feuilles à «froid» (30°C) au moyen d'un mélange $CH_3OH$-$H_2O$ (90:10) v/v, contient des flavonoïdes, principalement quatre, à savoir: la vitexine (avec un rendement de 0,081% en poids par rapport au poids des feuilles sèches de départ), l'isoorientine (avec un rendement de 0,066% en poids), la séquoiaflavone ou 7-O-méthylamentoflavone (avec un rendement de 0,052% en poids) et le vitexine-2″-O-acétyl-7-O-méthyléther, ledit article ne mentionnant pas les propriétés pharmacologiques dudit extrait hydroalcoolique total ni celles des quatre flavonoïdes.

La structure chimique de la séquoiaflavone, qui est une biflavone appartenant à la famille de l'amentoflavone et des analogues de celle-ci, est connue de l'ouvrage de H.D. LOCKSLEY «The Chemistry of Biflavonoid Compounds» pages 207-312, Springer-Verlag Vienne, New York (1973) - voir en particulier le tableau I page 299-.

On sait aussi que l'extrait de Ginkgo biloba commercialisé sous les dénominations de «TEBONIN» et de «TANAKAN» en tant qu'agent vasodilatateur renferme quatre biflavones à savoir la bilobetine, la ginkgetine, l'isoginkgetine et la sciadopitysine (voir l'ouvrage de LOCKSLEY précité pages 298, 299 et 303).

On connaît également (demande de brevet français FR-A 2 007 352 SCWABE) un procédé d'extraction à chaud de Ginkgo biloba pour obtenir un mélange de divers flavonoïdes.

On connaît enfin (demande de brevet français FR-A 2 268 518) un procédé de préparation d'extraits végétaux contenant un mélange de flavonoïdes. Selon ce document, l'extraction à chaud est déconseillée.

Selon l'invention on préconise un nouveau procédé de préparation d'un extrait de Brackenridgea zanguebarica différent de celui de E. Bombardelli en ce sens qu'il favorise l'obtention de séquoiaflavone et de vitexine-2″-O-acétyl-7-O-méthyléther par un choix particulier des modalités opératoires [extraction à «chaud» (à une température comprise entre 45 et 60°C) au moyen d'un solvant choisi parmi l'acétone, les mélanges acétone-eau et les mélanges alcool-eau compris entre (30:70) v/v et (50:50) v/v].

De façon inattendue et surprenante, l'extrait obtenu selon l'invention, qui est différent de l'extrait de Ginkgo biloba précité, présente l'avantage d'avoir des propriétés hypotensives et en particulier antihypertensives persistantes, d'une part, et des propriétés hémorhéologiques, d'autre part, que ne possède pas ledit extrait de Ginkgo biloba, la notice de la spécialité TANAKAN donnée dans le «Dictionnaire Vidal», page 1250, O.V.P., Paris (1982) signalant l'absence d'effet hypotenseur.

Un des buts de l'invention est de proposer un procédé permettant d'obtenir un extrait utile en thérapeutique et renfermant comme substance essentiellement active la séquoiaflavone, dont les propriétés pharmacologiques n'ont jamais été décrites.

La séquoiaflavone répond à la formule

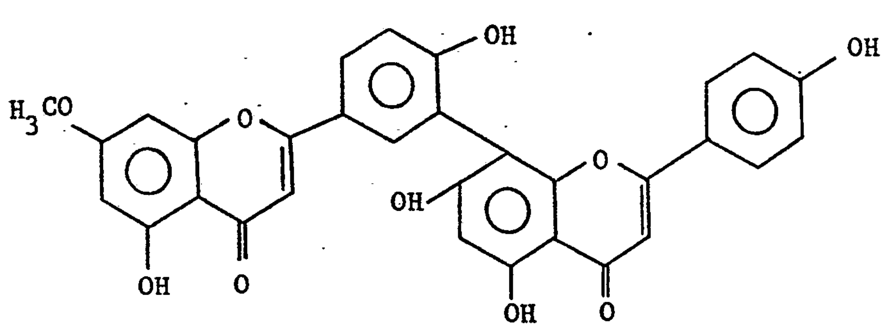

Un autre but de l'invention est de proposer un procédé d'obtention de la sequoiaflavone avec un rendement supérieur à celui du procédé décrit par E. BOMBARDELLI et al.

Dans ce qui suit, par «substance active», on entend le ou les produits contenus dans l'extrait selon l'invention agissant en tant que principe actif vasodilatateur antiagrégant plaquettaire, antithrombotique, tels que la sequioaflavone.

Le procédé selon l'invention pour la préparation d'un extrait de Brackenridgea zanguebarica, dans lequel on soumet les feuilles de la plante à une extraction avec deux solvants, est caractérisé en ce que

1°) l'on soumet les feuilles de Brackenridgea zanguebarica préalablement séchées et broyées à une extraction avec un solvant choisi parmi l'ensemble constitué par

a) l'acétone,

b) les mélanges acétone-eau ayant un rapport volumique $CH_3COCH_3$-$H_2O$ supérieur ou égal à (3:7) v/v, et

c) les mélanges alcool inférieur en $C_1$–$C_3$ – eau ayant un rapport volumique alcool – eau compris entre (3:7) v/v et (1:1) v/v,

à une température comprise entre 45 et 60°C; et

2°) l'on soumet l'extrait ainsi obtenu à un traitement choisi parmi l'ensemble constitué par

a) l'extraction au moyen d'un solvant choisi parmi l'ensemble comprenant les hydrocarbures aliphatiques en $C_1$-$C_4$ halogénes (tels que notamment le chloroforme, le chlorure de méthylène, le chlorure d'éthylène) et leurs mélanges, pour extraire la substance active soluble dans ledit solvant et écarter la plus grande partie des substances indésirables non solubles dans ledit solvant, et

b) le traitement au moyen d'un solvant choisi parmi l'ensemble comprenant les hydrocarbures aliphatiques en $C_5$–$C_7$, les hydrocarbures cycloaliphatiques en $C_5$–$C_6$, les hydrocarbures aromatiques (tels que notamment le pentane, l'hexane, l'heptane, le cyclopentane, le cyclohexane, le benzène, le toluène et le xylène) et leurs mélanges, pour écarter la plus grande partie des substances indésirables solubles dans ledit solvant, la substance active n'étant pas soluble dans ledit solvant; et,

3°) on concentre le milieu liquide résultant à une température inférieure ou égale à 50°C sous pression réduite.

De façon avantageuse on traitera au stade 1°) 1 kg de feuilles (préalablement séchées à l'étuve à 37–38°C et broyées) par 5 à 20 litres de solvant d'extraction (acétone, mélanges acétone-eau ou alcool-eau sus-visés). De façon avantageuse également, la concentration du stade 3°) est effectuée à une température inférieure ou égale à 50°C sous pression réduite, notamment de l'ordre de 10 à 50 mmHg (soit, avec 1 mmHg = $1{,}333224 \times 10^2$ pascals, une pression de l'ordre de $1{,}33 \times 10^3$ à $6{,}66 \times 10^3$ pascals), de façon à obtenir 0,002 à 0,250 litre de concentrat (liquide ou visqueux) à partir de 1 litre d'extrait liquide obtenu au stade 2°) et renfermant la substance active.

Le cas échéant, on peut prévoir une concentration de l'extrait liquide obtenu ou stade 1°) avant d'entreprendre le traitement du stade 2°). Une telle concentration quand elle est effectuée peut être réalisée selon les modalités du stade 3°).

L'extrait obtenu au stade 3°) peut être utilisé tel quel en thérapeutique sous une forme pharmaceutique appropriée. Il peut également être amené à l'état sec, notamment par lyophilisation, nébulisation ou dessication sous vide.

Le cas échéant, cet extrait peut également être purifié par chromatographie, avantageusement

(i) par chromatographie sur résine neutre (notamment une résine connue pour fixer les substances phénoliques telle que «AMBERLITE XAD4», «AMBERLITE XAD2» ou «DUOLITE S 761») fixant la substance active et ne retenant pas les substances indésirables encore présentes, puis lavage à l'eau et élution au moyen d'une alcool inférieur en $C_1$–$C_3$ pour recueillir la substance active qui est contenue dans l'éluat alcoolique; ou,

(ii) par chromatographie sur colonne de silice pour séparer, si nécessaire, les constituants de la substance active (notamment par élution au moyen d'un mélange choisi parmi les mélanges tels que $CHCl_3$–MeOH–$H_2O$, $CH_3CO_2$Et–MeOH–$H_2O$, $CH_3CO_2$Et–$H_2O$, polyamide-alcool).

La sequoiaflavone, qui est le produit le plus intéressant sur le plan thérapeutique de la substance active, peut être obtenue industriellement par cristallisation dans un alcool inférieur en $C_1$–$C_3$, le dioxanne, la pyridine ou l'acétonitrile à partir de l'extrait résultant du stade 3°) ou de l'extrait purifié par chromatographie comme illustré ci-après dans les exemples.

L'extrait obtenu selon le procédé de l'invention qui renferme de la sequoiaflavone, d'une part, et la sequoiaflavone pure obtenue par cristallisation, d'autre part, sont utiles en thérapeutique en tant qu'agents vasodilatateurs dans le traitement des maladies de la circulation artérielle, agents anti-agrégants plaquettaires et antithrombotiques. Ils présentent d'intéressantes activités vasotropes (associées à un effet inotrope positif) et hémorhéologiques.

Selon l'invention, on préconise donc:

- les médicaments contenant de la sequoiaflavone;
- et l'utilisation de l'Extrait de Brackenridgea zanguebarica tel qu'obtenu dans le procédé décrit-ci-dessus, pour la fabrication d'un médicament ayant un effet vasodilatateur, anti-agrégant plaquettaire et/ou anti-thrombotique.

D'autres avantages et caractéristiques de l'invention seront mieux compris à la lecture qui va suivre (i) d'exemples de préparation, et (ii) d'essais pharmacologiques.

Exemple 1

Préparation d'un extrait total de Brackenridgea zanguebarica

50 kg de feuilles de Brackenridgea zanguebarica finement broyées sont extraites dans un percolateur statique à une température de 50°C, par 4 fois 200 l du mélange EtOH–$H_2O$ (50:50) v/v.

Les extraits hydroalcooliques sont réunis, filtrés puis extraits en continu à l'hexane jusqu'à élimination totale des substances colorées. La phase hexane est éliminée et la phase hydroalcoolique est concentrée sous pression de 30 mm de Hg (soit environ $3{,}999 \times 10^3$ pascals) jusqu'à un volume de 50 l et à une température ne dépassant par 50°C.

La solution aqueuse obtenue est filtrée sur 0,5 kg de kieselguhr. Le filtrat est nébulisé (i.e. séché par projection) et on obtient 3,2 kg d'extrait sec, de couleur beige, soluble dans l'eau.

Exemple 2

Préparation d'un extrait purifié de Brackenridgea zanguebarica

50 kg de feuilles de Brackenridgea zanguebarica finement broyées sont extraites à faible reflux dans un percolateur statique par 5 fois 150 l du mélange $CH_3COCH_3$-$H_2O$ (40:60) v/v.

Les extraits hydroacétoniques réunis sont concentrés sous vide jusqu'à complète élimination du solvant puis le concentré obtenu est traité par 0,5 kg de kieselguhr puis filtré.

La phase aqueuse limpide est traitée sur une colonne de 80 l de Amberlite XAD4 activée, la vitesse d'élution étant réglée de telle sorte qu'il n'y ait aucune substance flavonoïdique dans l'éluat. On réalise ce contrôle sur plaque de gel de silice [éluant: acétate d'éthyle-éthanol-eau (100 13,5 10) v/v]. Les flavonoïdes sont révélés en développant la plaque à l'acide chlorosulfonique dans de l'acide acétique et en les examinant sous rayonnement ultra-violet en comparaison avec les substances de référence.

Lorsque l'adsorption est terminée la colonne est lavée par 100 l d'eau puis 150 l de méthanol. Par concentration à sec de l'éluat méthanolique on obtient 1,52 kg d'extrait enrichi en sequoiaflavone utile pour la préparation de formes pharmaceutiques liquides.

Exemple 3

Préparation de sequoiaflavone cristallisée

100 kg de feuilles de Brackenridgea zanguebarica finement broyées sont extraites par 4 fois 200 l d'acétone pure, pendant quatre heures à 50–55°C.

Les extraits acétoniques réunis sont concentrés sous pression normale jusqu'à obtention d'un volume de 100 l puis par la suite sous pression réduite, à une température ne dépassant par 50°C, jusqu'à consistance sirupeuse.

Le concentré est traité à faible reflux pendant 2 heures par 100 l d'hexane et le résidu insoluble est recueilli par filtration. Le solvant est évaporé et le résidu solide est repris par 10 l de méthanol puis laissé au repos pendant une nuit. On obtient 0,5 kg d'un mélange flavonoïdique cristallisé qui, une fois séché, est dissous dans 10 l de dioxanne à l'ébullition. La solution est traitée par 0,05 kg de charbon actif, filtrée et on laisse cristalliser à température ambiante pendant 24 heures. Après filtration et dessication sous vide à 50°C, on obtient 0,24 kg de sequoiaflavone ayant les caractéristiques suivantes:

F = 202–204°C

$E^{1\%}_{1\,cm}(270\text{ nm}) = 736$

Exemple 4

Préparatoin de sequoiaflavone et de vitexine-2″-O-acétyl-7-O-méthyléther

50 kg de feuilles de Brackenridgea zanguebarica finement broyées sont extraites 4 fois par 200 l du mélange MeOH-$H_2O$ (50:50) v/v à une température de 50°C. Les extraits réunis sont filtrés puis extraits par 3 fois 50 l de chloroforme.

On concentre sous vide la phase chloroformique, à une température ne dépassant par 50°C, jusqu'à un volume de 2,5 l. Le concentré chloroformique est chromatographié sur une colonne de 10 kg de gel de silice stabilisé par de l'acétate d'éthyle saturé d'eau. On élue la colonne à l'acétate d'éthyle saturé d'eau et on obtient successivement la sequoiaflavone puis la vitexine-2″-O-acétyl-7-O-méthyléther. Les deux fractions ainsi obtenues sont évaporées à sec séparément.

Par recristallisation de la sequoiaflavone dans le méthanol on obtient 0,130 kg de produit pur.

Par recristallisation de la vitexine-2″-O-acétyl-7-O-méthyléther dans l'acétate d'éthyle, on obtient 0,350 kg de produit ayant les caractéristiques suivantes:

$\alpha^{20°C}_{D} = -62,5°$ (C = 0,5; pyridine)

spectrométrie de masse: m/e = 488

F = 180°C

On a résumé ci-après les résultats des essais pharmacologiques qui ont été entrepris.

La sequoiaflavone obtenue selon l'exemple 3, administrée chez la souris par voie endopéritonéale et orale, à la dose de 3000 mg/kg n'a entraîné aucune mortalité, ni aucun signe de toxicité particulier. Ce produit, administré à la souris pendant cinq jours consécutifs, soit par voie orale, soit par voie intrapéritonéale, à la dose de 1000 mg/kg a été extrêmement bien toléré. En ce qui concerne l'activité hypotensive chez le rat génétiquement hypertendu, le produit a été administré par voie orale à raison de 1 à 4 mg/kg. Les résultats de cette expérimentation figurent dans le tableau I.

Pour étudier l'action de la sequoiaflavone obtenue selon l'exemple 3, sur l'irrigation cérébrale, on a déterminé chez la souris en condition d'anoxie (sous atmosphère comprenant en volume 97% de $N_2$ et 3% de $O_2$) les temps de survie en secondes. On constate que les animaux traités ont des temps de survie égaux à ceux obtenus avec les produits de référence tels que la vincamine et la papavérine, et significativement différents de ceux des témoins.

Dans ce qui suit on a résumé les résultats des essais entrepris pour comparer un extrait de Brakkenridgea zanguebarica selon l'invention renfermant 97,5% en poids de sequoiaflavone et désigné ci-après «produit IBLF» avec un produit de référence connu, l'extrait de Ginkgo biloba («produit A-1″) précité et commercialisé sous le nom de TANAKAN.

I - HYPOXIE AIGUE

A des lots de 20 souris mâle (chaque animal ayant un poids moyen de 22 g) on administre les produits à étudier (100 mg/kg) per os dans un véhicule gommeux (eau contenant 30 g/l de gomme arabique). Les animaux sont placés dans une atmosphère appauvrie en oxygène ($O_2$:5%; $N_2$:95%)

et on note le temps de survie. On constate que l'augmentation en pourcentage du temps de survie des animaux traités, par rapport au lot témoin recevant le véhicule seul, est de 32±28% pour A-1 et de 68±28% pour IBLF.

II - ISCHEMIE CEREBRALE

Sur des rats mâles Long Evans de 250 g, on pratique une ligature en 2 temps des artères carotides: clampage de l'artère carotide gauche, puis 24 h. plus tard, clampage de l'artère carotide «sans nom». Des lots de 10 animaux reçoivent pendant neuf jours soit un véhicule hydroalcoolique [EtOH–$H_2O$ (55:45) v/v] (lot témoin) soit les produits à tester (100 mg/kg) per os dans le même véhicule (lots traités), les clampages carotidiens intervenant les 2 derniers jours.

24 h. après le deuxième clampage vasculaire, on note le taux de survie des animaux. On observe que les animaux témoins ont un taux de survie de 25%, que les animaux ayant reçu A-1 ont un taux de survie de 33% et que les animaux ayant reçu IBLF ont un taux de survie de 50%.

III - ACTION ANTI-THROMBOSE PULMONAIRE

La technique utilisée est celle de UZUNOVA, Prostaglandins 13 (No 5) pages 995–1002 (1977). En bref on induit une thrombose pulmonaire chez la souris par injection d'acide arachidonique, en fait on induit une agrégation plaquettaire massive de telle façon que les agrégats plaquettaires formés sont retenus au niveau du lit pulmonaire et provoquent la mort des animaux. On évalue en conséquence le taux de survie des animaux ayant reçu l'agent agrégant.

On observe que à la dose de 100 mg/kg per os IBLF en suspension dans de l'eau gommeuse (eau contenant 30 g/l de gomme arabique) possède une activité antithrombotique pulmonaire (taux de survie de 22%), alors que A-1 dans les mêmes conditions n'a pas d'activité protectrice statistiquement significative.

On a par ailleurs étudié l'activité hémorhéologique. Cette étude a été conduite à l'aide de 2 techniques:

- technique I, filtration érythrocytaire selon la méthode de Reid et Dormandy modifiée,
- technique II, selon la méthode dite «pression de filtration», sur des érythrocytes dont la déformabilité a été préalablement altérée (par vieillissement accéléré), le IBLF et un produit de référence, la Pentoxifylline (ci-après «A-2»), étant étudiés à la concentration finale de 20 µg/ml.

On observe que IBLF améliore de façon statistiquement très significative la filtrabilité des hématies vieillies à 37°C quelle soit la technique utilisée.

technique I:
IBLF: + 92%; A-2 = 68%

technique II:
IBLF: +42%; A-2 = 16%.

En ce qui concerne l'activité antiagrégante plaquettaire, on a observé, selon la technique de BORN et al., J. Physiol. 168, 178 (1962), que IBLF inhibe totalement chez le rat mâle l'agrégation plaquettaire induite par l'acide arachidonique à la dose (concentration finale de 0,5 µg/ml.

En clinique, on a obtenu de bons résultats en administrant à l'homme un extrait selon l'invention renfermant de la sequoiaflavone ou constitué par la sequoiaflavone, à une dose quotidienne de 5 à 1000 mg par jour.

Tableau I

Activité sur la pression artérielle du rat génétiquement hypertendu

| Produit | Dose mg/kg per os | Nombre d'animaux | Pression artérielle systolique (mmHg) sur 9 Jours | | | | | | | |
|---|---|---|---|---|---|---|---|---|---|---|
| | | | 0 | 1 | 2 | 3 | 4 | 5 | 8 | 9 |
| Témoins | | 6 | 192±5 | 192±5 | 188±5 | 188±5 | 192±5 | 192±5 | 192±5 | 192±5 |
| Séquoiaflavone | 1 | 6 | 192±5 | 188±4 | 177±7 | 177±7 | 175±6 | 175±6 | 172±5* | 172±5* |
| obtenue selon | 2 | 6 | 203±8 | 192±5 | 193±4 | 190±6* | 185±8* | 183±9 | 183±9 | 180±10 |
| l'exemple 3 | 4 | 6 | 198±6 | 192±7 | 185±7* | 180±6* | 178±7* | 178±7* | 178±7* | 177±8* |

Note: * statistiquement significatif [$p < 0,05$ (t Student)]

Tableau II

Anoxie induite à l'azote chez la souris

| Traitement (a) | | Nombre d'animaux | Temps de survie en secondes (d) |
|---|---|---|---|
| Produit | Dose mg/kg I.P. | | |
| Témoin (b) | – | 10 | 340±33* |

Tableau II (suite)

Anoxie induite à l'azote chez la souris

| Traitement (a) Produit | Dose mg/kg I.P. | Nombre d'animaux | Temps de survie en secondes (d) |
|---|---|---|---|
| Sequoiaflavone | 2 | 10 | 552 ± 27* |
| Selon exemple 3 (c) | 8 | 10 | 578 ± 14* |

Notes:
(a) traitement effectué 1 heure avant la mise en anoxie
(b) recevant 50 g/l de gomme arabique à raison de 10 ml/kg
(c) dans de l'eau gommeuse comme les témoins
(d) moyenne ± écart standard à la moyenne
* statistiquement significatif [$p < 0{,}05$ (t Dunnett)]

Tableau III

Temps d'hémorragie

| Produit | Dose mg/kg per os | Temps d'hémorragie en secondes (a) (b) | | | |
|---|---|---|---|---|---|
| | | 2 heures | Nombre d'animaux | 4 heures | Nombre d'animaux |
| Témoins (a) (c) | – | 32,4 ± 2,09 | 10 | 36,4 ± 3,54 | 10 |
| Sequoiaflavone (d) | 2 | 61,9 ± 3,90** (+91,0%) | 10 | 49,8 ± 5,96 (+36,8%) | 10 |
| Sequoiaflavone (d) | 4 | 54,5 ± 5,59** (+68,2%) | 10 | 47,9 ± 6,29 (+31,6%) | 10 |

Notes:
(a) moyenne ± écart standard à la moyenne
(b) entre parenthèse, les variations en % par rapport aux témoins
(c) recevant de la gomme arabique à 50 g/l dans de l'eau
(d) selon Exemple 3, dans de l'eau gommeuse comme les témoins
** statistiquement significatif [$p < 0{,}01$ (t Dunnett)]

**Revendications**

1. Procédé de préparation d'un extrait de Brackenridgea zanguebarica comprenant de la quoiaflavone utile en thérapeutique, ledit procédé, qui met en œuvre l'utilisation de deux solvants d'extraction, étant caractérisé en ce que

1°) l'on soumet les feuilles de Brackenridgea zanguebarica préalablement séchées et broyées à une extraction avec un solvant choisi parmi l'ensemble constitué par

a) l'acétone,

b) les mélanges acétone-eau ayant un rapport volumique $CH_3COCH_3$-$H_2O$ supérieur ou égale à (3:7) v/v, et

c) les mélanges alcool inférieur en $C_1$–$C_3$ – eau ayant un rapport volumique alcool-eau compris entre (3:7) v/v et (1:1) v/v, à une température comprise entre 45 et 60°C;

2°) l'on soumet l'extrait ainsi obtenu à un traitement choisi parmi l'ensemble constitué par

a) l'extraction au moyen d'un solvant choisi parmi l'ensemble comprenant les hydrocarbures aliphatiques en $C_1$–$C_4$ halogénés et leurs mélanges, pour extraire la substance active soluble dans ledit solvant et écarter la plus grande partie des substances indésirables non solubles dans ledit solvant; et

b) le traitement au moyen d'un solvant choisi parmi l'ensemble comprenant les hydrocarbures aliphatiques en $C_5$–$C_7$, les hydrocarbures cycloaliphatiques en $C_5$–$C_6$, les hydrocarbures aromatiques et leurs mélanges, pour écarter la plus grande partie des substances indésirables solubles dans ledit solvant, la substance active n'étant pas soluble dans ledit solvant; et

3°) l'on concentre le milieu liquide résultant à une température inférieure ou égale à 50°C sous pression réduite

2. Procédé selon la revendication 1, caractérisé en ce que, au stade 1°), on traite 1 kg de feuilles de Brackenridgea zanguebarica, préalablement séchées à 37–38°C et broyées, par 5 à 20 litres de solvant d'extraction.

3. Procédé selon la revendication 1, caractérisé en ce que la concentration du stade 3°) est effectuée à une température inférieure ou égale à 50°C sous pression réduite de l'ordre de $1{,}33 \times 10^3$ à $6{,}66 \times 10^3$ pascals, de façon à obtenir 0,002 à 0,250 litre de concentrat à partir de 1 litre d'extrait

obtenu au stade 2°) et refermant la matière active.

4. Procédé selon la revendication 1, caractérisé en ce que, après le stade 1°) et avant d'entreprendre le stade 2°), on procède à une concentration sous pression réduite.

5. Procédé selon la revendication 1 caractérisé en ce que le produit obtenu au stade 3°) est soumis à une purification par chromatographie.

6. Procédé selon la revendication 5, caractérisé en ce que la purification est réalisée par chromatographie sur résine neutre fixant la substance active et ne retenant pas les substances indésirables encore présentes, puis lavage à l'eau et élution au moyen d'un alcool inférieur en $C_1$–$C_3$ pour recueillir la substance active qui est contenue dans l'éluat alcoolique.

7. Procédé selon la revendication 5, caractérisé en ce que la purification est réalisée par chromatographie sur colonne de silice pour séparer, si nécessiare, les constituants de la substance active (notamment par élution au moyen d'un mélange choisi parmi les mélanges tels que $CHCl_3$–$MeOH$–$H_2O$, $CH_3CO_2Et$–$MeOH$–$H_2O$, $CH_3CO_2Et$–$H_2O$, polyamide-alcool).

8. Procédé selon l'une quelconque des revendications 1, 6 et 7 pour la préparation de sequoiaflavone cristallisée à partir d'un extrait de Brackenridgea zanguebarica selon le stade 3°) le cas échéant purifié par chromatographie, caractérisé en ce que la substance active est soumise à une opération de recristallisation dans un solvant choisi parmi l'ensemble constitué par les alcools inférieurs en $C_1$–$C_3$, le dioxanne, la pyridine et l'acétonitrile.

9. Médicament, caractérisé en ce qu'il contient de la sequoiaflavone.

10. Utilisation d'un extrait de Brackenridgea zanguebarica pour la fabrication d'un médicament ayant un effet vasodilatateur anti-agrégant plaquettaire et/ou anti-thrombotique.

**Patentansprüche**

1. Verfahren zur Herstellung eines therapeutisch nützlichen Extraktes von Brackenridgea zanguebarica umfassend Sequoiaflavon, welches Verfahren, bei dem zwei Extraktionslösungsmittel zum Einsatz gelangen, dadurch gekennzeichnet ist, dass man

1) die zuvor getrockneten und gemahlenen Blätter von Brackenridgea zanguebarica einer Extraktion mit einem Lösungsmittel, ausgewählt aus der Gruppe bestehend aus

a) Aceton,

b) Mischungen Aceton-Wasser mit einem Volumsverhältnis $CH_3COCH_3$–$H_2O$ von höher oder gleich (3:7) v/v, und

c) Mischungen $C_1$–$C_3$-Niedrigalkohol-Wasser mit einem Volumsverhältnis Alkohol-Wasser zwischen (3:7) v/v und (1:1) v/v, bei einer Temperatur zwischen 45 und 60°C unterzieht;

2) den so erhaltenen Extrakt einer Behandlung unterzieht, ausgewählt aus der Gruppe bestehend aus

a) der Extraktion mit einem Lösungsmittel, ausgewählt aus der Gruppe umfassend halogenierte aliphatische $C_1$–$C_4$-Kohlenwasserstoffe und Mischungen davon, zum Extrahieren der in diesem Lösungsmittel löslichen aktiven Substanzen und zum Entfernen des grössten Teils der in diesem Lösungsmittel nicht löslichen unerwünschten Substanzen; und

b) der Behandlung mit einem Lösungsmittel, ausgewählt aus der Gruppe umfassend aliphatische $C_5$–$C_7$-Kohlenwasserstoffe, cycloaliphatische $C_5$–$C_6$-Kohlenwasserstoffe, aromatische Kohlenwasserstoffe und Mischungen davon zum Entfernen des grössten Teils der in diesem Lösungsmittel löslichen unerwünschten Substanzen, wobei die aktive Substanz in diesem Lösungsmittel nicht löslich ist; und

3) das resultierende flüssige Milieu bei einer Temperatur von weniger oder gleich 50°C unter reduziertem Druck konzentriert.

2. Verfahren nach Anspruch 1, dadurch gekennzeichnet, dass man in Stufe 1) 1 kg Blätter von Brackenridgea zanguebarica, die zuvor bei 37 bis 38°C getrocknet und gemahlen wurden, mit 5 bis 20 l Extraktionslösungsmittel behandelt.

3. Verfahren nach Anspruch 1, dadurch gekennzeichnet, dass die Konzentration der Stufe 3) bei einer Temperatur von unter oder gleich 50°C unter reduziertem Druck im Bereich von $1{,}33 \times 10^3$ bis $6{,}66 \times 10^3$ Pa derart durchgeführt wird, dass man 0,002 bis 0,250 l Konzentrat aus 1 l in Stufe 2) erhaltenem und das aktive Material enthaltendem Extrakt gewinnt.

4. Verfahren nach Anspruch 1, dadurch gekennzeichnet, dass man nach der Stufe 1) und vor Durchführung der Stufe 2) eine Konzentration unter reduziertem Druck vornimmt.

5. Verfahren nach Anspruch 1, dadurch gekennzeichnet, dass das in Stufe 3) erhaltene Produkt einer Reinigung mittels Chromatografie unterzogen wird.

6. Verfahren nach Anspruch 5, dadurch gekennzeichnet, dass die Reinigung mittels Chromatografie auf einem neutralen Harz, das die aktive Substanz fixiert und die noch vorhandenen unerwünschten Substanzen nicht hält, dann Waschen mit Wasser und Eluieren mit einem $C_1$–$C_3$-Niedrigalkohol zur Gewinnung der in dem Alkoholeluat enthaltenen aktiven Substanz erfolgt.

7. Verfahren nach Anspruch 5, dadurch gekennzeichnet, dass die Reinigung mittels Chromatografie auf einer Silicagelsäule notwendigenfalls zur Trennung der Bestandteile der aktiven Substanz (insbesondere durch Eluieren mittels einer Mischung, ausgewählt aus Mischungen, wie $CHCl_3$–$MeOH$–$H_2O$, $CH_3CO_2Et$–$MeOH$–$H_2O$, $CH_3CO_2Et$–$H_2O$, Polyamid-Alkohol) ausgeführt wird.

8. Verfahren nach einem der Ansprüche 1, 6 und 7 zur Herstellung von kristallisiertem Sequoiaflavon aus einem Extrakt von Brackenridgea zanguebarica nach Stufe 3), gegebenenfalls mittels Chromatografie gereinigt, dadurch gekennzeichnet, dass die aktive Substanz einem Umkristallisationsschritt in einem Lösungsmittel, ausgewählt aus der Gruppe bestehend aus $C_1$-$C_3$-Niedrigalkoholen, Dioxan, Pyridin und Acetonitril, unterzogen wird.

9. Medikament, dadurch gekennzeichnet, dass es Sequoiaflavon enthält.

10. Verwendung eines Extraktes von Brackenridgea zanguebarica zur Erzeugung eines Medikaments mit vasodilatatorischer plättchenaggregationshemmender und/oder antithrombotischer Wirkung.

**Claims**

1. Process for preparing an extract of Brackenridgea zanguebarica containing sequoiaflavone useful in therapeutics, said process, which involves the use of two extracting solvents, is characterized in that

1°) leaves of Brackenridgea zanguebarica dried and groud beforehand, are subjected to an extraction with a solvent selected from the group constituted by

a) acetone,

b) acetone-water mixtures having a volume ratio $CH_3COCH_3$-$H_2O$ higher than or equal to (3:7) v/v, and

c) $C_1$-$C_3$ lower alcohol-water mixtures having a volume ratio water-alcohol comprised between (3:7) v/v and (1:1) v/v, at a temperature comprised between 45 and 60°C;

2°) the extract thus obtained is subjected to a treatment selected from the group constituted by

a) the extraction by means of a solvent selected from the group comprising $C_1$-$C_4$ halogenated aliphatic hydrocarbons, and the mixtures thereof, in order to extract the active substance soluble in said solvent, and to remove the largest part of the indesirable substances non soluble in said solvent, and

b) the treatment by means of a solvent selected from the group comprising $C_5$-$C_7$ alipahtic hydrocarbons, $C_5$-$C_6$ cycloaliphatic hydrocarbons, aromatic hydrocarbons and the mixtures thereof, in order to remove the largest part of the indesirable substances in said solvent, the active substance being insoluble in said solvent; and

3) the resulting liquid medium is concentrated at a temperature lower than or equal to 50°C under reduced pressure.

2. Process according to claim 1, characterized in that, in step 1°), 1 kg of leaves of Brackenridgea zanguebarica, dried at 37-38°C and ground beforehand, is treated using 5 to 20 litres of extracting solvent.

3. Process according to claim 1, characterized in that the concentration of step 3°) is carried out at a temperature lower than or equal to 50°C under reduced pressure in the range of $1.33 \times 10^3$ to $6.66 \times 10^3$ pascals, so as to obtain 0.002 to 0.250 litre of concentrate from 1 litre of the extract obtained in step 2°) and containing the active substance.

4. Process according to claim 1, characterized in that, following step 1°) and before carrying out step 2°), a concentration under reduced pressure is carried out.

5. Process according to claim 1, characterized in that the product obtained to in step 3°) is subjected to a purification by chromatography.

6. Process according to claim 5, characterized in that the purification is carried out by chromatography on a neutral resin which fixes the active substance and does not retain the still present indesirable substances, then washing with water and elution by means of $C_1$-$C_3$ lower alkohol, to collect the active substance which is contained in the alkoholic eluant.

7. Process according to claim 5, characterized in that the purification is carried out by chromatography on a silica column in order to separate, if necessary, the constituents of the active substance (in particular by elution by means of a mixture selected from the mixtures such as $CHCl_3$-MeOH-$H_2O$, $CH_3CO_2$Et-MeOH-$H_2O$, $CH_3CO_2$Et-$H_2O$, polyamide-alkohol).

8. Process according to any one of claims 1, 6 and 7, for the preparation of sequoiaflavone crystallized from an extract of Brackenridgea zanguebarica according to step 3°), the extract being optionally purified by chromatography, characterized in that the active substance is subjected to an operation of recrystallization in a solvent selected from the group constituted by $C_1$-$C_3$ lower alkohols, dioxane, pyridine and acetonitrile.

9. Drug, characterized in that it contains sequoiaflavone.

10. Use of an extract of Brackenridgea zanguebarica for preparing a drug having a vasodilating, platelets anti-clotting and/or anti-thrombotic effect.